**Europäisches Patentamt**

**European Patent Office**

**Office européen des brevets**

(19)

(11) Veröffentlichungsnummer : **0 087 750 B2**

(12) # NEUE EUROPÄISCHE PATENTSCHRIFT

(45) Veröffentlichungstag der neuen Patentschrift :
**27.04.94 Patentblatt 94/17**

(51) Int. Cl.$^5$ : **C07K 5/06**, C07K 5/08,
A61K 37/02

(21) Anmeldenummer : **83101767.8**

(22) Anmeldetag : **23.02.83**

(54) **Glutaminhaltige Aminosäure-Zubereitungen.**

(30) Priorität : **25.02.82 DE 3206784**

(43) Veröffentlichungstag der Anmeldung :
**07.09.83 Patentblatt 83/36**

(45) Bekanntmachung des Hinweises auf die
Patenterteilung :
**19.06.85 Patentblatt 85/25**

(45) Bekanntmachung des Hinweises auf die
Entscheidung über den Einspruch :
**27.04.94 Patentblatt 94/17**

(84) Benannte Vertragsstaaten :
**AT CH DE GB LI SE**

(56) Entgegenhaltungen :
**WO-A-80/00216
BULLETIN OF THE CHEMICAL SOCIETY OF
JAPAN, Band 35, Nr. 12, 1962, Seiten
1966-1970, Chemical Society of Japan Y. SHI-
MONISHI et al.: "Studies on the synthesis of
peptides containing glutamine as the C-terminal. I. Protection of amide-nitrogen with
xanthyl group during peptide synthesis"**

(56) Entgegenhaltungen :
**CHEMICAL ABSTRACTS, Band 97, Nr. 21,
1982, Seite 146, Nr. 175470h, Columbus, Ohio,
USA D.C. PARISH et al.: "Isolation of glycylgutamine, the C-terminal dipeptide of the betaendorphin corticotropin prohormone"
BIOPOLYMERS, Band 11, 1972, Seiten
2493-2503, John Wiley & Sons, Inc. CHARLES
M. DEBER et al.: "Side chain interactions in
aromatic dipeptides"**

(73) Patentinhaber : **Kabi Pharmacia GmbH
Hofmannstrasse 26
D-91052 Erlangen (DE)**

(72) Erfinder : **Fürst, Peter, Prof. Dr.
Universität Hohenheim Garbenstrasse 30
D-7000 Stuttgart 70 (DE)**
Erfinder : **Pfaender, Peter, Prof. Dr.
Aichelestrasse 8
D-7000 Stuttgart 70 Plieningen (DE)**
Erfinder : **Fekl, Werner, Dr.
Rühlstrasse 1a
D-8520 Erlangen (DE)**

(74) Vertreter : **Wuesthoff, Franz, Dr.-Ing. et al
Wuesthoff & Wuesthoff Patent- und
Rechtsanwälte Schweigerstrasse 2
D-81541 München (DE)**

EP 0 087 750 B2

## Beschreibung

In vielen Fällen schwerer Erkrankung ist die Ernährung auf oralem Wege nicht oder nur schwer möglich. Insbesondere bei postoperativen und posttraumatischen Zuständen und in schweren Fällen von Karzinomen, Verbrennungen, Infektionen, akutem und chronischen Nieren-Versagen, Leberinsuffizienz sowie langanhaltender Bewußtlosigkeit oder schweren Stoffwechselstörungen müssen die Patienten dann parenteral mit Infusionslösungen ernährt werden, wobei zur Aufrechterhaltung der Ernährung dem Organismus neben Kohlenhydraten und Fett insbesondere Aminosäuren parenteral zugeführt werden müssen. Wegen der negativen Stickstoffbilanz, besonders bei postoperativer und posttraumatischer Stoffwechsellage, ist die Versorgung mit Aminosäuren von besonderer Bedeutung, wobei das Angebot nicht nur die essentiellen Aminosäuren umfassen soll, die der Organismus nicht selbst synthetisieren kann, sondern auch die anderen Aminosäuren, damit der Verlust an körpereigenen Proteinen möglichst schnell durch erneuten Aufbau dieser Proteine wieder ausgeglichen werden kann.

Zu den wichtigen Aminosäuren, die hierbei dem Organismus zur Verfügung gestellt werden müssen, gehört u. a. auch das Glutamin, dessen Konzentration im intrazellulären Aminosäuremuster bei der erwähnten Stoffwechsellage häufig einen besonders starken Abfall - bis zu 50% - zeigt (J. Askanazi et al, Ann. Surg., Bd. 191, S.465-472 (1980) und Ann. Surg., Bd. 192, S. 78-85 (1980)).

Ein besonderes Problem bei der parenteralen Zufuhr von Glutamin mittels aminosäurehaltigen Infusionslösungen liegt aber darin, daß diese Aminosäure ein toxisches Cyclisierungsprodukt bildet und daher zur intravenösen Anwendung nicht geeignet ist. Diese Umwandlung des Glutamins kann z. B. schon beim Hitzesterilisieren einer wäßrigen Lösung eintreten, oder auch im Organismus, wenn es als solches appliziert wird.

Es wurde nun erkannt, daß das Glutamin als an der $\alpha$-Aminogruppe durch $\alpha$-Aminoacylreste substituiertes Derivat, d. h. in Form der Tri- oder Dipeptide des Glutamins dem Organismus, insbesondere durch Infusionslösungen, zugeführt werden kann, ohne Gefahr der Bildung des toxischen Cyclisierungsprodukts. Diese Di- und Tripeptide des Glutamins mit anderen Aminosäuren, wie Glycin, Alanin, Leucin, Isoleucin oder Valin, sind sehr leicht wasserlöslich, so daß sie gegebenenfalls in relativ hoher Konzentration in den Infusionslösungen angewendet werden können, wobei gleichzeitig dem Organismus auch andere Aminosäuren zugeführt werden, ohne daß dadurch osmotische Problem auftreten. Die Löslichkeit beispielsweise des Gly-Ala-Gln beträgt 680 g/L $H_2O$, während die freien Aminosäuren eine geringere Löslichkeit besitzen: Glycerin: 225 g/L $H_2O$; Alanin: 158 g/L $H_2O$ und Glutamin: 325 g/L $H_2O$.

Nach der Infusion werden im Organismus diese Di- oder Tripeptide durch körpereigene Aminopeptidasen, z. B. durch die in Säugetiergeweben vorhandenen Peptidhydrolasen, aufgespalten und dadurch dann das Glutamin langsam freigelegt, zur unmittelbaren Verwertung beim Aufbau der körpereigenen Proteine. Die endständige Aminogruppe des Glutamins wird nämlich u.a. durch die Glutamin-Aminotransferase auf $\alpha$-Ketocarbonsäuren übertragen, weshalb für die körpereigene Synthese von Aminosäuren das Glutamin von großer Bedeutung ist. Durch die Aminoacylierung des Glutamins wird auch eine Stabilisierung der endständigen Amidgruppe erreicht, und zwar sowohl gegen übliche Hydrolyse bei der Sterilisierung der Lösungen als auch gegen die Glutaminase-Enzymreaktion im Orgnismus.

Erfindungsgemäß wird das Glutamin als $\alpha$-aminoacyliertes Derivat, d. h. also in Form der Di- oder Tripeptide, verwendet, wobei diese zusammen mit anderen Aminosäuren in den oralen Aminosäurepräparaten oder -infusionslösungen vorhanden sind. Hierdurch gelingt es, auch größere Mengen an Glutamin dem Organismus zuzuführen, ohne daß es zu toxischen Erscheinungen kommen kann. Vorzugsweise enthalten die Präparate, insbesondere die Infusionslösungen zur parenteralen Ernährung, als Dipeptid das Alanyl-L-Glutamin oder als Tripeptid das Glycyl-L-alanyl-L-glutamin. Die Infusionslösungen sollen die Di- oder Tripeptide des Glutamins in Mengen von 1-50 g/L enthalten und können gegebenenfalls weitere niedere Peptide enthalten sowie andere Nahrungsstoffe, wie Kohlenhydrate, z.B. Glucose, oder auch emulgierte Fette und Öle sowie Vitamine und Mineralsalze, wie sie üblicherweise in den Infusionslösungen zur parenteralen Ernährung vorhanden sind.

Es ist seit langem aus der Arbeit von Thierfelder und v. Cramm "Über glutaminhaltige Peptide und zur Frage ihres Vorkommens im Eiweiß" (Hoppe-Seylers, Bd. 105, Seiten 58 ff, 1919) bekannt, Glutamin durch Acylierung der $\alpha$-Aminogruppe gegen die Hydrolyse durch Salzsäure zu stabilisieren. Aus der PCT Anmeldung WO-A-80/00216 war u.a. bekannt, daß Peptide bestimmte Aminsäuren des Prolins, wie z.B. Prolyl-glutamin, die Verträglichkeit von Arzneimitteln auf Morphin-Basis verbessern können. Darüberhinaus war aus der Veröffentlichung von Adibi "Clearance of Dipeptides from Plasma; Role of Kidney and Intestine" in Peptide-Transport and Hydrolyses", Seite 265-285, Elsevier, Amsterdam 1977, bekannt, daß durch Hydrolasen in den Geweben des Organismus die Dipeptide Glycyl-glycin und Glycyl-leucin im Gegensatz zum Glycyl-sarcosin gut gespalten werden, so daß sie bei intravenöser Applikation schnell aus dem Plasma eliminiert werden.

Wenn auch das erfindungsgemäß anstelle des freien Glutamins zu verwendende gut wasserlösliche $\alpha$-aminoacylierte Glutamin vorzugsweise dem Organismus zusammen mit anderen Aminosäuren als Infusionslösung zugeführt werden soll, so eignen sich diese Glutaminderivate auch für die Applikation in oralen Aminosäure-Präparaten, die als Granulate oder in Dragee-Form eingenommen werden.

Die erfindungsgemäß zu verwendenden Di- oder Tripeptide des Glutamins, wie das L-alanyl-L-glutamin oder das Glycyl-L-alanyl-L-glutamin können leicht durch die N-Carboxy-Anhydrid-Methode hergestellt werden. Die Peptide werden hierbei vom Carboxy-Ende her aufgebaut, wobei die freie Aminogruppe der vorgelegten Aminosäure mit dem jeweiligen N-Carboxy-Anhydrid der anzuhängenden Aminosäure reagiert. Diese Methode ist durch R. Hirschmann et al in J. Org. Chem., Bd. 32, 3415-3425 (1967) und in J. Am. Chem. Soc., Bd. 93, 2746-2754 (1971) beschrieben worden. Sie wurde auch zur Herstellung von Alanyl-glutamin und anderen Peptiden angewendet, Biopolymers, Bd. 11, 2493-2503 (1972). Diese Methode läßt sich in automatischen Vorrichtungen durchführen, siehe P. Pfaender et al, Hoppe-Seyler's Z. Physiol-Chem ., Bd. 354, S. 267-285 (1973) und DE-OS 2 416 941 sowie US-PS 3 951 741.

Bei der bekannten Synthesemethode unter Verwendung der N-Carboxy-Anhydrid-Aminosäureverbindungen wird in Gegenwart eines Boratpuffers gearbeitet. Da die für therapeutische Zwecke notwendige Abtrennung des erhaltenen Tripeptids von dem Boratpuffer Schwierigkeiten bereitet, empfiehlt es sich, die Synthese in Aqua dest. durchzuführen, was überraschenderweise mit guten Ausbeuten gelingt. Um die Di- oder Tripeptide von Nebenprodukten, d.h. eventuell entstandenen höheren Peptiden abzutrennen, läßt sich z. B. das Alanyl-Glutamin-Dipeptid oder das Glycyl-Alanyl-Glutamin-Tripeptid leicht chromatographisch reinigen, z. B. unter Verwendung von Sephadex-Säulen.

Durch Auflösen der gereinigten Peptide und der gewünschten Aminosäuren in Aqua dest. werden Infusionslösungen erhalten, die gegebenenfalls noch Mineralsalze und andere Stoffe enthalten können und sich leicht isotonisch einstellen lassen. Die Lösungen können in üblicher Weise hitzesterilisiert werden (z. B. 0,5 h, 120°C, pH = 5,5-7,5), ohne Gefahr einer Veränderung des Tripeptids oder Dipeptids. Im Falle der Verwendung zusammen mit Glucose oder anderen Kohlenhydraten in Infusionslösungen für parenterale Ernährung sind diese jedoch erst nach der Sterilisierung oder kurz vor der Verwendung beim Patienten zuzufügen, um Nebenreaktionen der freien Aminogruppe des Peptids mit der Glucose bei der Hitzesterilisierung auszuschließen.

Nachfolgend sei die Herstellung des Ala-Gln-Dipeptids und des Gly-Ala-Gln-Tripeptids unter Anwendung der N-Carboxy-Anhydrid-Methode beschrieben. Diese Verbindungen können dann erfindungsgemäß für Infusionslösungen verwendet werden.

Herstellung von L-Alanin-L-Glutamin (Ala-Gln)

a) Herstellung von Alanin-N-Carboxy-Anhydrid (Ala-NCA)

In einem 2-L-Dreihals-Kolben wurden 30 g Alanin (L-Form) ($\triangleq 3.4 \times 10^{-2}$ Mol) in 1200 mL getrocknetem Tetrahydrofuran suspendiert. Unter Rühren wurde bei 30°C bis 40°C ein trockener Phosgen-Strom mit etwa 2 Blasen/sec eingeleitet. Nach 1-1,5 h war die Aminosäure bis auf einen kleinen Rest gelöst, worauf das Einleiten von Phosgen abgebrochen wurde, um eine Überphosgenierung zu vermeiden. Zur Vertreibung überschüssigen Phosgens wurde mit trockenem Stickstoff begast und anschließend das ungelöste Alanin abdekantiert und das Tetrahydrofuran am Rotationsverdampfer abgezogen. Der feste Rückstand wurde in 40 mL Aceton aufgenommen und mit einem Überschuß Petrolether gefällt. Es wurde unter $N_2$-Atmosphäre abgenutscht und das gereinigte Alanin-NCA im Exsikkator über $P_2O_5$ und NaOH-Plätzchen getrocknet. Es wurden Ausbeuten von ca. 85-89% erhalten.

b) Umsetzung von Ala-NCA mit Glutamin

5 g feinst gepulvertes L-Glutamin ($\triangleq 0,034$ Mol) wurden in 300 mL $H_2O$ bidest. gelöst. Nach dem Kühlen der Lösung auf 0°C und Einstellung der Lösung mit ca. 50% NaOH auf pH 10,2 wurden 4,26g Ala-NCA ($\triangleq 0,037$ Mol) innerhalb von 10 Min. in kleinen Anteilen zugegeben, um die pH-Schwankungen möglichst gering zu halten. Danach wurde mit ca. 50% $H_2SO_4$ auf pH 3,5 titriert, um das Peptidcarbaminat zu decarboxylieren. Anschließend wurde die Lösung neutralisiert und lyophilisiert. Die Rohausbeute betrug 14,0 g.

Das Lyophilisat des Rohansatzes (14,0 g) wurde auf 3 Sephadex-G-10-Läufe (jeweils ca. 4,7 g) aufgeteilt, um die Kapazität der Säule nicht zu überschreiten. Bereits beim 1. Lauf wurde eine reine Dipeptidfraktion erhalten. Die unreinen Fraktionen wurden erneut auf die Sephadex-Säule gegeben und so weiteres reines Dipeptid erhalten, dessen Reinheit dünnschicht-chromatographisch an Kieselgelplatten mit ButanolEisessig-Wasser (4 : 1 : 1) als Laufmittel und mit Ninhydrin als Entwicklungsreagens kontrolliert wurde. Das erhaltene Ala-Gln kann den Aminosäurelösungen in gewünschter Menge zugesetzt werden.

Herstellung von Glycin-L-Alanin-L-Glutamin (Gly-Ala-Gln)

a) Herstellung von Glycin-N-Carboxy-Anhydrid (Gly-NCA)

In entsprechender Weise, wie bei der Herstellung von Ala-NCA beschrieben, wurde auch das Glycin-N-Carboxy-Anhydrid hergestellt, wobei aber die Ausbeuten, bezogen auf das eingesetzte Glycin, geringer waren.

b) Umsetzung des Ala-Gln mit Gly-NCA

4,34 g ($\triangleq$ 0,02 Mol) des Ala-Gln-Dipeptids wurden in 300 mL $H_2O$ bidest. mit 2,22 g ($\triangleq$ 0,022 Mol) NCA-Gly unter Verwendung der in der DEOS 2 416 941 und US-PS 3 951 741 beschriebenen Vorrichtung umgesetzt. Das durch Lyophilisation erhaltene rohe Gly-Ala-Gln wurde auf der Sephadex-G-10-Säule gereinigt, wobei die Fraktionen wieder dünnschicht-chromatographisch kontrolliert wurden. Das erhaltene Gly-Ala-Gln eignet sich für die Verwendung in Infusionslösungen.

Die erfindungsgemäße Verwendung der Di- und Tripeptide des Glutamins als Glutaminquelle in Aminosäure-Infusionslösungen wird im folgenden anhand einiger Beispiele gezeigt. Die dabei zusätzlich zu den üblichen Aminosäuren verwendeten Tripeptide des Cystins bzw. Tyrosins zeichnen sich durch gute Wasserlöslichkeit aus und können nach der N-Carboxy-Anhydrid-Methode hergestellt werden, was in einer gleichzeitig eingereichten Anmeldung näher erläutert wird.

Beispiel 1

Gly-Ala-Gln und die anderen Substanzen wurden in den angegebenen Mengen in Aqua dest. gelöst, die Lösung in Infusionsflaschen abgefüllt und dann sterilisiert. Die Lösung kann in einer Menge von 10-30 mL je kg Körpergewicht und Tag infundiert werden.

| Peptide und Aminosäuren | g/1000 mL |
|---|---|
| Gly-Ala-Gln | 22,5 |
| L-Isoleucin | 2,1 |
| L-Leucin | 2,9 |
| L-Lysin-acetat | 4,37 |
| L-Methionin | 1,80 |
| L-Phenylalanin | 1,25 |
| L-Threonin | 2,70 |
| L-Tryptophan | 1,00 |
| L-Valin | 2,30 |
| L-Arginin | 7,00 |
| L-Histidin | 1,75 |
| L-Alanin | 0,7 |
| L-Glutaminsäure | 5,00 |
| Glycin | 0,8 |
| L-Prolin | 7,0 |
| L-Serin | 6,2 |
| CyS-Lys (CyS)[1] | 2,0 |
| Tyr-Lys (Tyr)[2] | 2,0 |
| Kaliumhydroxid | 1,96 |
| Natriumchlorid | 2,57 |
| Calciumglycero-phosphat-2-hydrat | 0,49 |
| Magnesiumchlorid | 0,61 |
| Natriumglycerophosphat | 5,51 |
| Kaliumchlorid | 0,37 |

[1] $N^2$-Cystinyl-$N^6$-cystinyl-lysin
[2] $N^2$-Tyrosinyl-$N^6$-tyrosinyl-lysin

Die beiden Tripeptide enthalten L-Cystin bzw. L-Tyrosin in leicht löslicher Form.

Beispiel 2

Die Substanzen wurden wie in Beispiel 1 beschrieben in Aqua dest. gelöst und in Infusionsflaschen abgefüllt und sterilisiert.

| Peptide und Aminosäuren | g/1000 mL |
|---|---|
| Gly-Ala-Gln | 1,9 |
| L-Isoleucin | 7,60 |
| L-Leucin | 8,50 |
| L-Lysin-Monomalat | 7,67 |
| L-Methionin | 0,25 |
| L-Phenylalanin | 0,10 |
| L-Threonin | 1,20 |
| L-Tryptophan | 0,10 |
| L-Valin | 6,40 |
| L-Arginin | 4,90 |
| L-Histidin | 0,60 |
| L-Ornithin-L-aspartat | 8,03 |
| L-Alanin | 1,50 |
| L-Glutaminsäure | 1,00 |
| Glycin | 0,20 |
| L-Prolin | 1,20 |
| L-Serin | 1,75 |
| CyS-Lys (CyS) | 0,30 |
| Tyr-Lys (Tyr) | 0,20 |
| Natriumglycerophosphat-5-hydrat | 4,52 |
| Magnesiumchlorid | 1,02 |
| Kaliumchlorid | 1,34 |

Beispiel 3

Die Substanzen wurden wie in Beispiel 1 beschrieben in Aqua dest. gelöst und in Infusionsflaschen abgefüllt und sterilisiert.

| Peptide und Aminosäuren | g/1000 mL |
|---|---|
| Gly-Ala-Gln | 11,3 |
| L-Isoleucin | 2,51 |
| L-Leucin | 2,79 |
| L-Lysin | 1,45 |
| L-Methionin | 0,80 |
| L-Phenylalanin | 1,40 |
| L-Threonin | 1,74 |
| L-Tryptophan | 0,56 |
| L-Valin | 2,09 |
| L-Arginin | 3,49 |
| L-Histidin | 0,70 |
| L-Alanin | 0,50 |
| L-Asparaginsäure | 4,00 |
| L-Glutaminsäure | 3,20 |
| Glycin | 0,50 |
| L-Prolin | 3,80 |
| CyS-Lys (CyS) | 2,00 |
| Tyr-Lys (Tyr) | 2,00 |
| Nikotinamid | 0,06 |
| Pyridoxinhydrochlorid | 0,04 |
| Riboflavin-5-phosphat-Na-salz | 0,0025 |
| Kaliumhydroxid | 1,40 |
| Natriumhydroxid | 1,20 |
| Calciumchlorid | 0,735 |
| Magnesiumacetat | 0,536 |

## Patentansprüche

1. Verwendung von Glutamin als α-aminoacyliertes Derivat in Form der wasserlöslichen Di- oder Tripeptide zur Herstellung von wäßrigen glutaminhaltigen Aminosäurezubereitungen für die orale oder parenterale Ernährung zum Aufbau oder zur Erhaltung körpereigenen Proteins.

2. Verwendung der Di- oder Tripeptide nach Anspuch 1 in einer Menge von 1 bis 50 g je Liter wäßriger Zubereitung.

3. Verwendung des Dipeptids L-Alanyl-L-glutamin oder des Tripeptids Glycyl-L-alanyl-L-glutamin nach Anspruch 1 oder 2.

## Claims

1. Use of glutamine as an α-aminoacylated derivative in the form of the water-soluble dipeptides or tripeptides, to produce aqueous, glutamine containing amino acid preparations for the oral or parenteral feeding for the elaboration or conservation of body protein.

2. Use of the dipeptides or tripeptides according to claim 1 in a proportion of 1 to 50 g per litre of aqueous preparation.

3. Use of the dipeptide L-alanyl-L-glutamine or of the tripeptide glycyl-L-alanyl-L-glutamine according to claim 1 or 2.

## Revendications

1. Utilisation de la glutamine en tant que dérivé α-aminoacylé sous la forme des dipeptides ou des tripeptides solubles dans l'eau en vue de la production de préparations d'aminoacides aqueuses, contenant de la glutamine et destinées à l'alimentation orale ou parentérale afin de l'élaboration ou de la conservation de la protéine propre du corps.

2. Utilisation des dipeptides et des tripeptides selon la revendication 1 dans une quantité allant de 1 à 50 g par litre d'une préparation aqueuse.

3. Utilisation du dipeptide L-alanyl-L-glutamine ou du tripeptide glycyl-L-alanyl-L-glutamine selon la revendication 1 ou 2.